# FASCICULE DE BREVET EUROPEEN

(11) **EP 4 077 701 B1**
(45) Date de publication et mention de la délivrance du brevet: **08.05.2024**
(21) Numéro de dépôt: 20848715.7
(22) Date de dépôt: 17.12.2020
(51) Int. Cl.: C12P 19/26, C08B 37/08

(54) **PROCÉDÉ D'OBTENTION DE CHITINE ET/OU DE CHITOSAN METTANT EN OEUVRE DEUX HYDROLYSES ENZYMATIQUES**
VERFAHREN ZUR HERSTELLUNG VON CHITIN UND / ODER CHITOSAN UNTER VERWENDUNG VON ZWEI ENZYMATISCHEN HYDROLYSEN
METHOD FOR OBTAINING CHITIN AND/OR CHITOSAN USING TWO ENZYMATIC HYDROLYSES

(30) Priorité: 19.12.2019 FR 1915001
(43) Date de publication de la demande: 26.10.2022
(73) Titulaire: Ynsect, 91058 Évry-Courcouronnes Cedex (FR)
(72) Inventeur: SANCHEZ, Lorena, 91260 Juvisy-sur-Orge (FR); HUSSET, Valère, 78340 Les Clayes sous Bois (FR); KIRECHE, Adam, 94500 Champigny-sur-Marne (FR)
(74) Mandataire: Santarelli
(86) Numéro de dépôt international: PCT/FR2020/052517
(87) Numéro de publication internationale: WO 2021/123654

(56) Documents cités:
- WO-A1-2016/108033
- WO-A1-2019/115970

## Description

La présente invention concerne un procédé d'obtention de chitine à partir de cuticules d'insectes.

Par « chitine », on entend un polymère composé majoritairement d'unités glucosamine et N-acétyl-glucosamine, lesdites unités pouvant être optionnellement substituées par des acides aminés et/ou des peptides.

Il est connu que la chitine est synthétisée par de nombreuses espèces du monde vivant, et notamment par les insectes puisqu'elle constitue 3 à 60% de leur exosq uelette.

La cuticule est la couche externe (ou exosquelette) sécrétée par l'épiderme des insectes. Elle est en général formée de trois couches : l'épicuticule, l'exocuticule et l'endocuticule.

Les insectes constituent une matière première renouvelable et abondante, il est donc très avantageux d'en extraire la chitine.

La chitine est également connue pour être une source de chitosan, obtenu après désacétylation de celle-ci.

Par « chitosan », on entend selon la présente invention les produits de désacétylation de la chitine. La limite usuelle entre le chitosan et la chitine est déterminée par le degré d'acétylation : un composé ayant un degré d'acétylation inférieur à 50% est nommé chitosan, au-delà, un composé ayant un degré d'acétylation supérieur à 50% est nommé chitine.

La chitine et le chitosan possèdent de nombreuses applications, notamment dans le domaine alimentaire, cosmétique ou médical.

La chitine est habituellement obtenue par un procédé chimique ou enzymatique, ces procédés ayant pour but d'éliminer les protéines liées à la chitine. L'utilisation d'un procédé enzymatique est particulièrement avantageuse puisqu'il permet notamment de garder une structure de la chitine au plus proche de sa structure originelle.

Les documents WO 2019/115970 et WO 2016/108033 se rapportent à des procédés d'obtention de chitine à partir d'insectes comprenant une étape d'hydrolyse enzymatique par une protéase.

Toutefois, les procédés enzymatiques usuellement employés impliquent un long temps de réaction et ne permettent pas de déprotéiniser la chitine de manière satisfaisante.

Le travail des inventeurs a permis de développer un procédé d'extraction enzymatique palliant à ces inconvénients.

Ainsi, la présente invention concerne un procédé d'obtention de chitine à partir de cuticules d'insectes comprenant :
- une première hydrolyse enzymatique des cuticules d'insectes mettant en oeuvre au moins une endopeptidase,
- la séparation des cuticules hydrolysées résultant de la première hydrolyse enzymatique, du milieu d'hydrolyse,
- une seconde hydrolyse enzymatique des cuticules hydrolysées mettant en oeuvre au moins une endopeptidase, à l'exclusion d'exopeptidase.

Par « insectes », on entend des insectes à n'importe quel stade de développement, tel qu'un stade adulte, larvaire ou un stade de nymphe.

Avantageusement, les insectes préférés selon l'invention sont les coléoptères, les diptères, les lépidoptères, les orthoptères, les hyménoptères, les dictyoptères regroupant notamment les blattoptères, y inclus isoptères et les mantoptères, les phasmoptères, les hémiptères, les hétéroptères, les éphéméroptères et les mécoptères, ou leurs mélanges, de préférence, les coléoptères, les diptères, les lépidoptères, les orthoptères ou leurs mélanges, plus préférentiellement, les coléoptères.

Préférentiellement, les diptères appartiennent au sous-ordre des Brachycera.

Préférentiellement, les lépidoptères appartiennent au sous-ordre des Ditrysia, plus préférentiellement à la super-famille des Pyraloidea.

Préférentiellement, les coléoptères appartiennent à l'infra-ordre des Cucujiformia, en particulier aux familles des Tenebrionidae, Coccinellidae, Cerambycidae, Dryophthoridae ou leurs mélanges.

Plus préférentiellement, les coléoptères sont choisis parmi Tenebrio molitor, Alphitobius diaperinus, Zophobas morio, Tenebrio obscurus, Tribolium castaneum, Rhynchophorus ferrugineus et leurs mélanges, encore plus préférentiellement Tenebrio molitor.

Avantageusement, les cuticules d'insectes sont obtenues à partir du stade larvaire des espèces d'insectes visées ci-avant.

Par « hydrolyse enzymatique », on entend une réaction chimique dans laquelle il y a rupture d'une liaison chimique par l'eau, cette rupture étant catalysée par une enzyme ou composition enzymatique.

Selon l'invention, l'enzyme ou composition enzymatique a une activité protéolytique (protéase).

Par « peptidase » ou « protéase », on entend une enzyme capable de cliver une liaison peptidique d'une chaîne peptidique.

Les noms ou suffixes « protéase » et « peptidase » sont utilisés indifféremment tout au long de la présente demande.

Par « endopeptidase », on entend une peptidase capable de cliver une liaison peptidique interne à une chaîne peptidique, et qui agit donc préférentiellement loin d'une liaison peptidique terminale d'une chaîne peptidique (c'est-à-dire localisée à une extrémité C- ou N-terminale de la chaîne peptidique).

Par « exopeptidase », on entend une enzyme dont le mécanisme d'action permet de cliver uniquement une liaison peptidique terminale d'une chaîne peptidique, conduisant à la libération d'un acide aminé, d'un dipeptide ou d'un tripeptide.

Il arrive que les enzymes disponibles dans le commerce ne soient pas pures. Le choix d'une enzyme implique ainsi la présence d'autres enzymes (en quantité moindre) ayant le même type d'activité (par exemple d'autres types d'endopeptidase) ou même des activités différentes (par exemple, des exopeptidases, des lipases).

De ce fait, selon l'invention, par « endopeptidase » ou « exopeptidase », on entend que l'enzyme principale est, respectivement, une endopeptidase ou une exopeptidase. Par « enzyme principale », on vise l'enzyme dont l'activité est recherchée. Plus particulièrement, cette activité est déclarée par le fabricant dans le produit commercial. Cette déclaration peut notamment s'effectuer par une indication sur les fiches de données (sécurité, commerciale) associées au produit.

Il peut également arriver que le produit soit une composition enzymatique, c'est-à-dire un mélange de plusieurs enzymes principales. Dans un tel cas, chacune des enzymes est déclarée par le fabricant dans le produit commercial.

Avantageusement, la au moins une endopeptidase mise en oeuvre dans la première et la seconde hydrolyse enzymatique du procédé selon l'invention est une enzyme ou une composition enzymatique protéolytique, identique ou différente, la présence d'enzymes principales (déclarées par le fabricant) autres que protéolytiques étant exclue, afin de favoriser l'activité protéolytique.

Avantageusement, la au moins une endopeptidase mise en oeuvre dans la première hydrolyse enzymatique du procédé selon l'invention est choisie parmi une endopeptidase, un mélange d'endopeptidases et un mélange d'exopeptidase(s) et d'endopeptidase(s), de préférence, parmi une endopeptidase et un mélange d'endopeptidases, plus préférentiellement, est une endopeptidase.

Avantageusement, la au moins une endopeptidase mise en oeuvre dans la première hydrolyse est choisie parmi les endopeptidases à sérine (EC/IUB 3.4.21.XX), les endopeptidases à cystéine (EC/IUB 3.4.22.XX), les protéases aspartiques (EC/IUB 3.4.23.XX), les métalloendopeptidases (EC/IUB 3.4.24.XX) et les endopeptidases à thréonine (EC/IUB 3.4.25.XX).

De préférence, la au moins une endopeptidase mise en oeuvre dans la première hydrolyse est choisie parmi les endopeptidases à sérine, les endopeptidases à cystéine, les protéases aspartiques et les métalloendopeptidases, plus préférentiellement, les endopeptidases à sérine et les métalloendopeptidases, encore plus préférentiellement, les endopeptidases à sérine.

Lorsque la au moins une endopeptidase, mise en oeuvre lors de la première hydrolyse uniquement, est une composition enzymatique sous forme d'un mélange d'exopeptidase(s) et d'endopeptidase(s), l' (les) exopeptidase(s) est (sont) choisie(s) parmi les aminopeptidases (EC/IUB 3.4.11.XX), les dipeptidases (EC/IUB 3.4.13.XX), les di- ou tripeptidyl peptidases (EC/IUB 3.4.14.XX), les peptidyl dipeptidases (EC/IUB 3.4.15.XX), les sérine carboxypeptidases (EC/IUB 3.4.16.XX), les métallocarboxypeptidases (EC/IUB 3.4.17.XX), les cystéine carboxypeptidases (EC/IUB 3.4.18.XX) et les oméga peptidases (EC/IUB 3.4.19.XX).

De préférence, l' (les) exopeptidase(s) pouvant être mise(s) en oeuvre dans la première hydrolyse est (sont) choisie(s) parmi les aminopeptidases, les di- ou tripeptidyl peptidases et les sérine carboxypeptidases, plus préférentiellement, les aminopeptidases.

A titre d'exemple, lors de la première hydrolyse enzymatique, lorsque la au moins une endopeptidase est une composition enzymatique, qui est un mélange d'exopeptidase(s) et d'endopeptidase(s), l'endopeptidase est telle qu'indiquée ci-avant.

De préférence, la au moins une endopeptidase est alors un mélange d'une endopeptidase à sérine, d'une protéase aspartique et d'une aminopeptidase.

Par « séparation du milieu d'hydrolyse », on vise plus particulièrement le fait d'isoler et de récupérer les cuticules hydrolysées.

Avantageusement, la séparation des cuticules hydrolysées résultant de la première hydrolyse enzymatique est effectuée par centrifugation, décantation ou filtration, de préférence par filtration.

Avantageusement, cette filtration est réalisée à l'aide d'un filtre Büchner, d'un filtre presse, d'un filtre à bandes ou d'un filtre à tamis, de préférence, à l'aide d'un filtre Büchner avec un maillage métallique.

De préférence, la taille des pores ou mailles du filtre utilisé lors de cette filtration est comprise entre 4 µm et 800 µm, plus préférentiellement, entre 40 µm et 550 µm, encore plus préférentiellement entre 140 µm et 530 µm, telle que par exemple 160 µm.

On notera que dans le cadre de la présente demande, et sauf stipulation contraire, les gammes de valeurs indiquées s'entendent bornes incluses.

Il est entendu que lors de la séparation des cuticules hydrolysées, la au moins une endopeptidase (en tant qu'enzyme ou composition enzymatique), mise en oeuvre lors de la première hydrolyse enzymatique est évacuée avec le milieu d'hydrolyse ; elle n'est donc pas ou très peu présente lors de la seconde hydrolyse enzymatique.

La seconde hydrolyse enzymatique met en oeuvre au moins une endopeptidase, à l'exclusion d'exopeptidase.

De même, par « au moins une endopeptidase, à l'exclusion d'exopeptidase », on entend une endopeptidase telle que définie ci-avant, avec la condition que la seconde hydrolyse ne comporte pas d'exopeptidase, c'est-à-dire que la au moins une endopeptidase est une endopeptidase ou est une composition enzymatique ne comportant pas d'exopeptidase.

De préférence, la au moins une endopeptidase mise en oeuvre lors de cette seconde hydrolyse enzymatique est une endopeptidase ou un mélange d'endopeptidases, plus préférentiellement, une endopeptidase.

Plus particulièrement, dans le procédé selon l'invention, la au moins une endopeptidase mise en oeuvre lors de la seconde hydrolyse enzymatique est une endopeptidase ou un mélange d'endopeptidases choisie(s) parmi les endopeptidases à sérine, les endopeptidases à cystéine, les protéases aspartiques, les métalloendopeptidases et les endopeptidases à thréonine.

De préférence, la au moins une endopeptidase mise en oeuvre lors de la seconde hydrolyse enzymatique est une endopeptidase ou un mélange d'endopeptidases choisie(s) parmi les endopeptidases à sérine, les endopeptidases à cystéine, les protéases aspartiques et les métalloendopeptidases.

Plus préférentiellement, la au moins une endopeptidase mise en oeuvre lors de la seconde hydrolyse enzymatique est une endopeptidase ou un mélange d'endopeptidases choisie(s) parmi les endopeptidases à sérine et les métalloendopeptidases.

En particulier, dans le procédé selon l'invention, la au moins une endopeptidase mise en oeuvre lors de la seconde hydrolyse enzymatique est une endopeptidase à sérine ou un mélange d'endopeptidases à sérine, et/ou une métalloendoprotéase ou un mélange de métalloendoprotéases, chaque endopeptidase à sérine étant choisie parmi le groupe constitué par la subtilisine et la trypsine, chaque métalloendoprotéase étant choisie parmi le groupe constitué par la bacillolysine et la thermolysine.

Plus particulièrement, la au moins une endopeptidase mise en oeuvre lors de la seconde hydrolyse enzymatique est plus préférentiellement une endopeptidase à sérine et/ou une métalloendoprotéase, l'endopeptidase à sérine étant la subtilisine, et la métalloendoprotéase étant la bacillolysine.

Enfin, encore plus préférentiellement, la au moins une endopeptidase mise en oeuvre lors de la seconde hydrolyse enzymatique est une endopeptidase choisie parmi les endopeptidases à sérine.

Les endopeptidases mises en oeuvre lors de la première et de la seconde étape d'hydrolyse peuvent être identiques, telles que par exemple, une même endopeptidase à sérine ou une même métalloendoprotéase.

Selon un mode de réalisation particulier, la au moins une endopeptidase mise en oeuvre lors de la première hydrolyse enzymatique comprend une endopeptidase à sérine, préférentiellement, est une endopeptidase à sérine, et la au moins une endopeptidase mise en oeuvre lors de la seconde hydrolyse enzymatique est également une endopeptidase à sérine, le groupe des endopeptidases à sérine étant avantageusement tel que décrit ci-après.

Dans le procédé selon l'invention, les endopeptidases à sérine mises en oeuvre dans l'une quelconque des étapes d'hydrolyse sont avantageusement choisies parmi le groupe constitué de la subtilisine (EC/IUB 3.4.21.62), la chymotrypsine (EC/IUB 3.4.21.1), la trypsine (EC/IUB 3.4.21.4), l'oryzine (EC/IUB 3.4.21.63), la glutamyl endopeptidase (EC/IUB 3.4.21.19), la streptogrisine A (EC/IUB 3.4.21.80) et/ou la streptogrisine B (EC/IUB 3.4.21.81), de préférence, de la subtilisine, la trypsine et/ou l'oryzine, plus préférentiellement, de la subtilisine et/ou la trypsine, encore plus préférentiellement, de la subtilisine.

De même, les endopeptidases à cystéine mises en oeuvre dans l'une quelconque des étapes d'hydrolyse sont avantageusement choisies parmi le groupe constitué de la bromélaïne (EC/IUB 3.4.22.32) et/ou la papaïne (EC/IUB 3.4.22.2), de préférence, de la bromélaïne.

De même, les protéases aspartiques mises en oeuvre dans l'une quelconque des étapes d'hydrolyse sont avantageusement l'aspergillopepsine I (EC/IUB 3.4.23.18).

De même, les métalloendoprotéases mises en oeuvre dans l'une quelconque des étapes d'hydrolyse sont avantageusement choisies parmi le groupe constitué de la bacillolysine (EC/IUB 3.4.24.28), la thermolysine (EC/IUB 3.4.24.27), la collagénase I (EC/IUB 3.4.24.3), la vibriolysine (EC/IUB 3.4.24.25), la pseudolysine (EC/IUB 3.4.24.26), l'auréolysine (EC/IUB 3.4.24.29), la coccolysine (EC/IUB 3.4.24.30), la mycolysine (EC/IUB 3.4.24.31), la métalloendopeptidase bêta-lytique (EC/IUB 3.4.24.32), la deutérolysine (EC/IUB 3.4.24.39) et/ou la serralysine (EC/IUB 3.4.24.40), de préférence, de la bacillolysine, la thermolysine et/ou la collagénase I, plus préférentiellement, de la bacillolysine et/ou la thermolysine, encore plus préférentiellement, de la bacillolysine.

Avantageusement, les aminopeptidases mises en oeuvre dans la première étape d'hydrolyse sont la leucyl aminopeptidase (EC/IUB 3.4.11.1).

Avantageusement, la au moins une endopeptidase mise en oeuvre lors de l'une quelconque des étapes d'hydrolyse enzymatique du procédé selon l'invention est d'origine bactérienne, fongique, animale et/ou végétale, préférentiellement d'origine bactérienne et/ou fongique, plus préférentiellement, d'origine bactérienne.

A titre d'exemple, la au moins une endopeptidase d'origine végétale est la bromélaïne ou la papaïne, préférentiellement, la bromélaïne. En particulier, la au moins une endopeptidase d'origine végétale peut être issue d'Ananas comosus ou de Carica papaia.

A titre d'exemple, la au moins une endopeptidase d'origine animale est la trypsine et/ou la chymotrypsine, plus préférentiellement, la trypsine. En particulier, la au moins une endopeptidase d'origine animale peut être issue de pancréas de porc (sus scrofa domesticus).

A titre d'exemple, la au moins une endopeptidase d'origine fongique est l'orizyne et/ou la leucyl aminopeptidase et/ou l'aspergillopepsine I, préférentiellement, l'oryzine ou un mélange d'orizyne, de leucyl aminopeptidase et d'aspergillopepsine I, plus préférentiellement, un mélange d'orizyne, de leucyl aminopeptidase et d'aspergillopepsine I. En particulier, la au moins une endopeptidase d'origine fongique peut être issue d'Aspergillus, plus particulièrement d'Aspergillus oryzae ou de Streptomyces, plus particulièrement de Streptomyces griseus, de préférence, d'Aspergillus oryzae.

A titre d'exemple, la au moins une endopeptidase d'origine bactérienne est la subtilisine, la bacillolysine, la thermolysine, la collagénase I ou un de leurs mélanges, préférentiellement, la subtilisine et/ou la bacillolysine, plus préférentiellement, la subtilisine. En particulier, la au moins une endopeptidase d'origine bactérienne peut être issue de Bacillus ou de Clostridium histolyticum, préférentiellement, de Bacillus licheniformis, Bacillus subtilis, Bacillus amyloliquefaciens ou Bacillus stearothermophilus, plus préférentiellement, de Bacillus licheniformis.

En particulier, dans le procédé selon l'invention, la au moins une endopeptidase mise en oeuvre lors de la seconde hydrolyse enzymatique est d'origine bactérienne.

Avantageusement, les enzymes ou composition enzymatique du procédé selon l'invention sont choisies parmi les enzymes commerciales du Tableau 1 suivant :

**[Tableau 1]**

| **Nom commercial** | **Origine** | **Espèce** | **Enzyme** | **Fournisseur** |
|---|---|---|---|---|
| Promod 439L | Bactérienne | *Bacillus licheniformis* | Subtilisine (CAS 9014-01-1)*, EC/IUB 3.4.21.62 | Biocatalysts |
| Novo-Pro D | | | Subtilisine*, EC/IUB 3.4.21.62* | Novozymes |
| Novozym 37071 | | | Subtilisine*, EC/IUB 3.4.21.62* | Novozymes |
| Alcalase 2.5 L PF | | | Subtilisine*, EC/IUB 3.4.21.62* | Novozymes |
| Corolase 7089 | | *Bacillus subtilis* | Métallo et sérine protéase* EC/IUB 3.4.2X.XX* | AB Enzymes |
| Sumizyme BNP-L | | | EC/IUB 3.4.2X.XX* | Shin Nihon Chemical |
| Food Pro PNL | | *Bacillus amyloliquefaciens* | Bacillolysine*, EC/IUB 3.4.24.28 | Dupont Danisco |
| Protin SD-NY100 | | | Bacillolysine, EC/IUB 3.4.24.28* | Amano Enzyme |
| Corolase 2TS | | *Bacillus stearothermophilus* | Thermolysine, EC/IUB 3.4.24.27* | AB Enzymes |
| Collagenase Type I | | *Clostridium histolyticum* | Collagénase (CAS 9001-12-1)*, EC/IUB 3.4.24.3 | Alfa Aesar |
| Promod 648L | Bactérienne et végétale | Mélange de souches, principalement *Bacillus* | Subtilisine, (CAS 9014-01-1)*, EC/IUB 3.4.21. 62; Papaïne, (CAS 9001-73-4)*, EC/IUB 3.4.22.2 | Biocatalysts |
| Prolyve NP conc | Fongique | *Aspergillus oryzae* | Oryzine, EC/IUB 3.4.21.63* | Soufflet Biotechnologies |
| FoodPro 51FP | | | Aspergillopepsine I*, EC/IUB 3.4.23.18 | Dupont Danisco |
| Sumizyme LP | | | Leucyl aminopeptidase, EC/IUB 3.4.11.1*; Oryzine, EC/IUB 3.4.21.63*; Aspergillopepsine I, EC/IUB 3.4.23.18* | Shin Nihon Chemical |
| Protease type XIV | | *Streptomyces griseus* | S. griseus Protease A*, EC/IUB 3.4.21.80; S. griseus Protease B*, EC/IUB 3.4.21.81; S. griseus Trypsin*; Aminopeptidase* | Sigma Aldrich |
| Pancréatine | Animale | *Sus scrofa domesticus* | Trypsine*, EC/IUB 3.4.21.4; Amylase* ; Lipase* ; Ribonucléase* ; Protéase* | Sigma-Aldrich |
| Bromélaïne de tige d'ananas | Végétale | *Ananas comosus* | Bromélaïne, EC/IUB 3.4.22.32* | Sigma-Aldrich |
| Lypaïne 48000 | | *Carica papaïa* | Papaïne, EC/IUB 3.4.22.2* | Soufflet Biotechnologies |

| | | | | |
|---|---|---|---|---|
| *Déclarée par le fournisseur | | | | |

De préférence, la au moins une endopeptidase mise en oeuvre dans la première hydrolyse du procédé selon l'invention est choisie parmi Promod 439L, Novo-Pro D, Novozym 37071, Alcalase 2.5 L PF et Sumizyme LP, plus préférentiellement, Promod 439L, Alcalase 2.5 L PF et Sumizyme LP.

De préférence, la au moins une endopeptidase mise en oeuvre dans la seconde hydrolyse du procédé selon l'invention est choisie parmi Promod 439L, Novo-Pro D, Novozym 37071 et Alcalase 2.5 L PF, plus préférentiellement, Promod 439L et Alcalase 2.5 L PF.

Selon un mode de réalisation particulièrement préféré, la au moins une endopeptidase mise en oeuvre lors de la première hydrolyse enzymatique est d'origine bactérienne ou fongique, et la au moins une endopeptidase mise en oeuvre lors de la seconde hydrolyse enzymatique est d'origine bactérienne.

Dans ce mode de réalisation, chaque au moins une endopeptidase mise en oeuvre lors des étapes d'hydrolyse est avantageusement une de celles décrites ci-avant.

Ainsi, selon ce mode de réalisation, la au moins une endopeptidase mise en oeuvre lors de la première hydrolyse enzymatique est de préférence la subtilisine, ou un mélange d'orizyne, de leucyl aminopeptidase et d'aspergillopepsine I, ou la bacillolysine, plus préférentiellement, la subtilisine, et la au moins une endopeptidase mise en oeuvre lors de la seconde hydrolyse enzymatique est de préférence la subtilisine ou la bacillolysine, plus préférentiellement, la subtilisine.

Une inactivation thermique de la au moins une endopeptidase (enzyme ou composition enzymatique) mise en oeuvre dans les étapes d'hydrolyse du procédé selon l'invention peut être effectuée suite à l'une quelconque des étapes d'hydrolyse enzymatique. Avantageusement, une seule inactivation thermique est effectuée, suite à la seconde hydrolyse enzymatique.

De préférence, cette inactivation thermique est effectuée en chauffant le milieu d'hydrolyse entre 80°C et 105°C, tel que par exemple à 85°C, pendant 10 à 30 minutes, plus préférentiellement, pendant 15 à 20 minutes.

Avantageusement, le milieu réactionnel obtenu à l'issue de la seconde hydrolyse enzymatique du procédé selon l'invention est chauffé à une température supérieure à 70°C, de préférence, supérieure à 75°C, plus préférentiellement, supérieure à 80°C, telle que par exemple 85°C, afin d'inactiver la au moins une endopeptidase.

Avantageusement, le procédé selon l'invention comprend en outre une étape de broyage des cuticules d'insectes préalable à la première hydrolyse enzymatique.

De préférence, les cuticules d'insectes sont broyées en présence d'eau, plus préférentiellement dans un ratio massique des cuticules par rapport à l'eau compris entre 1:5 et 1:7, tel que par exemple 1:6.

Tout au long de la présente demande, l'eau est de préférence de l'eau du robinet.

De préférence, l'étape de broyage permet de réduire la taille des cuticules d'insectes à une taille comprise entre 0,5 mm et 10 mm, plus préférentiellement, entre 1,0 et 7 mm, encore plus préférentiellement, entre 1,0 et 5 mm, telle que par exemple entre 1,0 et 2,0 mm.

Cette étape de broyage permet notamment d'améliorer l'efficacité des étapes d'hydrolyse et donc, de diminuer la durée de ces étapes.

Avantageusement, l'étape de broyage des cuticules d'insectes est réalisée à l'aide d'un hachoir à viande, d'un broyeur à marteaux, d'un moulin à lames, d'un broyeur à couteaux et/ou d'un disperseur, préférentiellement à l'aide d'un broyeur à couteaux tel que par exemple un Thermomix.

Avantageusement, les cuticules d'insectes sont lavées à l'eau après l'étape de broyage et avant la première hydrolyse enzymatique. Cette étape permet d'améliorer l'élimination des protéines qui ne sont pas liées aux cuticules.

Avantageusement, ces cuticules d'insectes lavées sont récupérées par une filtration, de préférence, à l'aide d'un filtre Büchner, d'un filtre presse, d'un filtre à bandes ou d'un filtre à tamis, de préférence, à l'aide d'un filtre Büchner avec un maillage métallique.

De préférence, la taille des pores ou mailles de ce filtre est comprise entre 4 µm et 800 µm, plus préférentiellement, entre 40 µm et 550 µm, encore plus préférentiellement entre 140 µm et 530 µm.

Avantageusement, les cuticules lavées sont pressées après leur récupération par une étape de filtration afin d'éliminer l'excès d'eau. De préférence, lesdites cuticules sont pressées jusqu'à obtenir une teneur en matière sèche comprise entre 35 et 65% en poids, plus préférentiellement, entre 45 et 55% en poids, sur le poids total des cuticules.

L'homme du métier sait quel type de presse utiliser pour cette étape de pressage, par exemple la presse Angelia 7500 de Angel.

Les cuticules d'insectes pressées peuvent être congelées avant l'étape de première hydrolyse enzymatique.

Avantageusement, les cuticules d'insectes du procédé selon l'invention sont obtenues par une étape de séparation des cuticules de la partie molle des insectes.

Par « partie molle », on vise la chair (comportant notamment les muscles et les viscères) et le jus (comportant notamment les liquides biologiques, l'eau et l'hémolymphe) des insectes. En particulier, la partie molle ne consiste pas en le jus des insectes.

Cette étape de séparation des cuticules de la partie molle est généralement précédée d'une étape d'abattage des insectes, qui peut avantageusement s'effectuer par choc thermique, tel que par ébouillantage ou par blanchiment.

Pour l'ébouillantage, les insectes, de préférence des larves, sont ainsi ébouillantés à l'eau pendant 2 à 20 minutes, préférentiellement, 5 à 15 minutes.

De préférence, l'eau est à une température comprise entre 87 à 100°C, préférentiellement 92 à 95°C.

La quantité d'eau introduite lors de l'ébouillantage est déterminée de la façon suivante : le ratio du volume d'eau en ml sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

Pour le blanchiment, les insectes, de préférence des larves, sont blanchis à l'eau ou à la vapeur (buses ou lit de vapeur) à une température comprise entre 80 et 105°C, de préférence entre 87 et 105°C, plus préférentiellement entre 95 et 100°C, encore plus préférentiellement 98°C ou bien à l'eau à une température comprise entre 90 et 100°C, préférentiellement entre 92 et 95°C (par buses d'aspersion) ou en mode mixte (eau + vapeur) à une température comprise entre 80 et 130°C, de préférence entre 90 et 120°C, plus préférentiellement entre 95 et 105°C, encore plus préférentiellement 98°C. Lorsque les insectes sont blanchis uniquement à la vapeur, le blanchiment est avantageusement réalisé dans des blancheurs à vapeur à circulation forcée (« forced steaming »). Le temps de séjour dans la chambre de blanchiment est compris entre 5 secondes et 15 minutes, préférentiellement entre 1 et 7 minutes.

Avantageusement, suite à l'étape d'abattage, les insectes sont directement utilisés pour la mise en oeuvre de l'étape de séparation des cuticules de la partie molle des insectes, c'est-à-dire que les insectes ne sont soumis à aucun traitement tel qu'un broyage, une congélation ou une déshydratation, entre ces deux étapes.

De préférence, cette étape de séparation des cuticules de la partie molle des insectes est réalisée à l'aide d'un filtre presse ou d'un séparateur à bande, plus préférentiellement, à l'aide d'un séparateur à bande.

Par « séparateur à bande », on vise un dispositif qui comporte une bande de serrage (ou bande presseuse) et un tambour perforé.

La bande de serrage permet l'apport et l'application des insectes contre le tambour perforé de sorte à faire passer, par pression, la partie molle des insectes à travers les perforations du tambour, tandis que les cuticules restent à l'extérieur du tambour.

Les cuticules peuvent ensuite être récupérées à l'aide d'un couteau racleur puis optionnellement congelées.

A titre d'exemple, on peut citer les séparateurs à bande provenant de la société Baader, tels que les séparateurs à bande 601 à 607 (« soft separator 601 to 607 »), ou encore les séparateurs à bande SEPAmatic^{®} de BFD Corporation (gamme 410 à 4000V).

Avantageusement, le diamètre des perforations du tambour est compris entre 0,5 et 3 mm, de préférence entre 1 et 2 mm.

Concernant la pression, l'homme du métier est à même de déterminer la pression à exercer permettant la séparation des cuticules de la partie molle des insectes.

Cette étape de séparation des insectes se distingue d'un pressage classique pouvant être réalisé par exemple avec une presse mono-vis ou bi-vis en ce qu'elle permet une séparation (nette) de la partie molle et des cuticules des insectes et non une séparation d'un jus d'une fraction solide.

En particulier, la séparation des cuticules de la partie molle s'effectue sans qu'aucune étape préalable de broyage des insectes, notamment sous forme de particules, n'ait été effectuée.

Avantageusement, la durée totale des étapes d'hydrolyse du procédé selon l'invention est inférieure à 6 h.

Par « durée totale des étapes d'hydrolyse », on entend la somme des temps de réaction de la première et de la seconde étape d'hydrolyse.

Typiquement, pour chaque hydrolyse, le temps de réaction de l'hydrolyse peut être défini comme le temps écoulé entre l'ajout de la au moins une endopeptidase dans le milieu d'hydrolyse et l'étape de séparation du milieu d'hydrolyse des cuticules hydrolysées, ou l'inactivation du milieu d'hydrolyse.

De préférence, la durée totale des étapes d'hydrolyse est comprise entre 2 h et 5 h 30, plus préférentiellement, entre 3 h et 5 h, encore plus préférentiellement 4 h.

Avantageusement, la durée de chaque étape d'hydrolyse est comprise entre 30 min et 4 h, de préférence, entre 1 h et 3 h, plus préférentiellement, entre 1 h 30 et 2 h 30, telle que par exemple égale à 2 h.

Avantageusement, la quantité totale desdites au moins une endopeptidase mises en oeuvre dans le procédé selon l'invention est comprise entre 10 U et 500 U par gramme de cuticules d'insectes, en poids sec.

Il est entendu que, dans tout le texte de la demande, « U », aussi appelé unité enzymatique, représente la quantité d'enzyme nécessaire pour libérer une micromole de tyrosine (provenant de la caséine) en une minute, à 37°C et à pH 7,5.

Par « quantité totale desdites au moins une endopeptidase », on entend la somme des quantités des au moins une endopeptidase introduites lors de la première et de la seconde hydrolyse enzymatique du procédé selon l'invention, ces quantités étant exprimées en unités enzymatiques telles que déterminées dans le paragraphe ci-avant.

De préférence, la quantité totale desdites au moins une endopeptidase mises en oeuvre dans le procédé selon l'invention est comprise entre 10 U et 500 U, plus préférentiellement, entre 20 U et 150 U, encore plus préférentiellement, entre 30 U et 60 U, telle que par exemple 40 U, par gramme de cuticules d'insectes, en poids sec.

Avantageusement, le ratio quantité de la au moins une endopeptidase mise en oeuvre lors de la première hydrolyse en U par gramme de cuticules d'insectes en poids sec / quantité de la au moins une endopeptidase mise en oeuvre lors de la seconde hydrolyse en U par gramme de cuticules d'insectes en poids sec est compris entre 0,5 et 10, de préférence, entre 0,8 et 5, plus préférentiellement, entre 1 et 2, tel que par exemple d'environ 1.

Avantageusement, la température de chaque hydrolyse enzymatique est comprise entre 35 et 70°C, de préférence, entre 45 et 60°C.

Le Tableau 2 résume l'origine ainsi que les activités enzymatiques observées pour les enzymes commerciales du Tableau 1. De plus, il y est indiqué la température d'hydrolyse enzymatique particulièrement préférée associée à chaque enzyme lors de son utilisation dans le procédé selon l'invention.

**[Tableau 2]**

| Enzyme | Activité enzymatique (U/g ou U/mL d'enzyme) | Température préférée (°C) |
|---|---|---|
| Promod 439L | 588,4 | 57 |
| Novo-Pro D | 1771,8 | 60 |
| Novozym 37071 | 2031 | 60 |
| Alcalase 2.5 L PF | 908,1 | 55 |
| Corolase 7089 | 1052,3 | 55 |
| Sumizyme BNP-L | 1152 | 55 |
| Food Pro PNL | 473,5 | 50 |
| Protin SD-NY100 | 4248,5 | 50 |
| Corolase 2TS | 918,1 | 60 |
| Collagenase Type I | 522,3 | 40 |
| Promod 648L | 577,4 | 60 |
| Prolyve NP conc | 1020,7 | 45 |
| FoodPro 51 FP | 751,7 | 50 |
| Sumizyme LP | 372,2 | 45 |
| Protease type XIV | 5098,6 | 40 |
| Pancréatine | 1236,4 | 45 |
| Bromélaïne (tige) | 851,7 | 45 |
| Lypaïne 48000 | 734,1 | 60 |

Avantageusement, les étapes d'hydrolyse enzymatique du procédé selon l'invention sont effectuées en présence d'eau à un ratio eau en g / cuticules en g et en poids sec compris entre 5 et 50, de préférence, entre 10 et 40, plus préférentiellement, entre 15 et 30, encore plus préférentiellement, entre 20 et 25, tel que par exemple de l'ordre de 22.

Avantageusement, le pH des étapes d'hydrolyse enzymatique est compris entre 6 et 9, préférentiellement entre 6,5 et 7,5, plus préférentiellement, entre 6,8 et 7,3.

Avantageusement, le procédé selon l'invention comprend en outre une étape de récupération des cuticules hydrolysées résultant de la seconde hydrolyse enzymatique. De préférence, cette étape de récupération est effectuée par centrifugation, décantation ou filtration, plus préférentiellement, par filtration.

De préférence, cette étape de filtration est réalisée à l'aide d'un filtre Büchner, d'un filtre presse, d'un filtre à bandes ou d'un filtre à tamis, plus préférentiellement, à l'aide d'un filtre Büchner avec un maillage métallique.

De préférence, la taille des pores ou mailles du filtre utilisé lors de cette filtration est comprise entre 4 µm et 800 µm, plus préférentiellement, entre 40 µm et 550 µm, encore plus préférentiellement entre 140 µm et 530 µm.

L'invention vise donc plus particulièrement un procédé particulier d'obtention de chitine à partir de cuticules d'insectes comprenant :
- une étape de séparation des cuticules de la partie molle des insectes,
- une étape de broyage des cuticules d'insectes,
- une première hydrolyse enzymatique des cuticules d'insectes broyées mettant en oeuvre au moins une endopeptidase,
- la séparation des cuticules hydrolysées résultant de la première hydrolyse enzymatique, du milieu d'hydrolyse,
- une seconde hydrolyse enzymatique des cuticules hydrolysées mettant en oeuvre au moins une endopeptidase, à l'exclusion d'exopeptidase,
- une inactivation thermique desdites au moins une endopeptidase mises en oeuvre,
- une étape de récupération des cuticules hydrolysées résultant de la seconde hydrolyse enzymatique.

Les étapes de ce procédé particulier sont avantageusement telles que décrites ci-avant, y inclus les modes de réalisation. Notamment, la au moins une endopeptidase mise en oeuvre lors de la première hydrolyse est une endopeptidase à sérine, une métalloendoprotéase ou un mélange d'une endopeptidase à sérine, d'une protéase aspartique et d'une aminopeptidase, et la au moins une endopeptidase mise en oeuvre lors de la seconde hydrolyse est notamment une endopeptidase à sérine ou une métalloendoprotéase.

Les cuticules hydrolysées résultant de la seconde hydrolyse enzymatique sont de la chitine. Cependant, la pureté de cette chitine peut être insatisfaisante pour certaines applications.

De ce fait, afin d'obtenir une chitine purifiée, il peut être avantageux que le procédé selon l'invention comprenne en outre, à l'issue de la seconde hydrolyse, une étape de traitement chimique.

Avantageusement, le traitement chimique est réalisé à l'aide d'une base forte.

Avantageusement, la base forte est choisie parmi l'hydroxyde de sodium ou soude, de potassium et d'ammonium. De préférence, la base forte est l'hydroxyde de sodium ou de potassium, plus préférentiellement l'hydroxyde de sodium.

De préférence, la base mise en oeuvre pour le traitement chimique est sous forme d'une solution basique aqueuse. Dans ce cas, la concentration molaire de la base en solution aqueuse est avantageusement comprise entre 0,1 et 4 mol.L⁻¹, de préférence comprise entre 0,5 et 2 mol.L⁻¹, encore plus préférentiellement égale à 1 mol.L⁻¹.

Le traitement chimique est avantageusement réalisé pendant une durée comprise entre 5 et 60 heures, de préférence pendant une durée comprise entre 15 et 25 heures, telle que par exemple 19 heures.

Ainsi, le procédé selon l'invention, en permettant de diminuer la quantité résiduelle de protéines à la surface des cuticules hydrolysées, a également l'avantage de diminuer la durée du traitement chimique.

Avantageusement, le traitement basique est réalisé à une température comprise entre 70 et 120°C, de préférence comprise entre 80 et 100°C, plus préférentiellement de l'ordre de 90°C.

Avantageusement, le procédé selon l'invention comprend en outre une étape de récupération de la chitine purifiée résultant de l'étape de traitement chimique. De préférence, cette étape de récupération est effectuée par centrifugation, décantation ou filtration, plus préférentiellement, par filtration.

De préférence, cette étape de filtration est réalisée à l'aide d'un filtre Büchner, d'un filtre presse, d'un filtre à bandes ou d'un filtre à tamis, plus préférentiellement, à l'aide d'un filtre à tamis.

De préférence, la taille des pores ou mailles du filtre utilisé lors de cette filtration est comprise entre 4 µm et 800 µm, plus préférentiellement, entre 40 µm et 550 µm, encore plus préférentiellement, entre 140 µm et 530 µm.

Optionnellement, le procédé selon l'invention comprend en outre une étape de lavage de la chitine purifiée.

Cette étape optionnelle de lavage de la chitine purifiée est avantageusement réalisée à l'aide d'eau du robinet, de préférence tiède, c'est-à-dire dont la température est comprise entre 15 et 60°C.

De préférence, le lavage de la chitine purifiée est réalisé jusqu'à neutralisation du pH.

Optionnellement, le procédé selon l'invention comprend en outre une étape de séchage de la chitine purifiée.

Cette étape optionnelle de séchage de la chitine purifiée est avantageusement réalisée à une température comprise entre 40 et 105°C, de préférence à environ 60°C.

L'étape de séchage de la chitine purifiée est avantageusement réalisée pendant une durée comprise entre 10 et 80 heures, de préférence pendant environ 24 heures.

Avantageusement, le séchage de la chitine purifiée est réalisé à l'aide d'une étuve de séchage, telle que le modèle FED 115 ou FP53 de la société Binder^{®}.

L'invention concerne également un procédé d'obtention de chitosan comportant un procédé d'obtention de chitine selon l'invention, tel qu'exposé ci-avant, et comprenant en outre une étape de désacétylation de la chitine.

Avantageusement, la désacétylation de la chitine est réalisée à l'aide d'une base forte.

De préférence, la base forte est choisie parmi l'hydroxyde de sodium ou soude, de potassium et d'ammonium. Plus préférentiellement, la base forte est l'hydroxyde de sodium ou de potassium, encore plus préférentiellement l'hydroxyde de sodium.

De préférence, la base utilisée pour le traitement basique est sous forme d'une solution basique aqueuse, de préférence une solution basique aqueuse concentrée.

Dans ce cas, la concentration molaire de la base en solution aqueuse est avantageusement comprise entre 6 et 25 mol.L⁻¹, préférentiellement comprise entre 8 et 19 mol.L⁻¹, plus préférentiellement comprise entre 10 et 19 mol.L⁻¹, encore plus préférentielle comprise entre 12 et 19 mol.L⁻¹.

De préférence, la désacétylation est réalisée pendant 1 à 24 heures et préférentiellement 2 à 18 heures.

Avantageusement, cette désacétylation est réalisée en deux fois, avec une étape intermédiaire de neutralisation du pH. Par exemple, la désacétylation pourra être réalisée en deux fois deux heures, c'est-à-dire pendant 4 heures, avec une étape intermédiaire de neutralisation du pH intermédiaire.

La température de désacétylation se situe avantageusement entre 80 et 150°C, de préférence entre 90 et 120°C et plus préférentiellement à 100°C.

Avantageusement, le procédé d'obtention de chitosan selon l'invention comprend en outre une étape de récupération du chitosan. De préférence, cette étape de récupération est effectuée par centrifugation, décantation ou filtration, plus préférentiellement, par filtration.

De préférence, cette étape de filtration est réalisée à l'aide d'un filtre Büchner, d'un filtre presse, d'un filtre à bandes ou d'un filtre à tamis, plus préférentiellement, à l'aide d'un filtre à tamis.

De préférence, la taille des pores ou mailles du filtre utilisé lors de cette filtration est comprise entre 4 µm et 800 µm, plus préférentiellement, entre 40 µm et 550 µm, encore plus préférentiellement entre 140 µm et 530 µm.

Optionnellement, le procédé d'obtention de chitosan selon l'invention comprend en outre une étape de lavage du chitosan.

Cette étape optionnelle de lavage du chitosan est avantageusement réalisée à l'aide d'eau du robinet, de préférence tiède, c'est-à-dire dont la température est comprise entre 15 et 60°C.

De préférence, le lavage du chitosan est réalisé jusqu'à neutralisation du pH.

Optionnellement, le procédé d'obtention de chitosan selon l'invention comprend en outre une étape de séchage du chitosan.

Cette étape optionnelle de séchage du chitosan est avantageusement réalisée à une température comprise entre 40 et 105°C, de préférence à environ 60°C.

L'étape de séchage du chitosan est avantageusement réalisée pendant une durée comprise entre 10 et 80 heures, de préférence pendant environ 24 heures.

Avantageusement, le séchage du chitosan est réalisé à l'aide d'une étuve de séchage, telle que le modèle FED 115 ou FP53 de la société Binder^{®}.

La présente invention est illustrée, de manière non limitative, par les exemples ci-après ainsi que les figures suivantes :

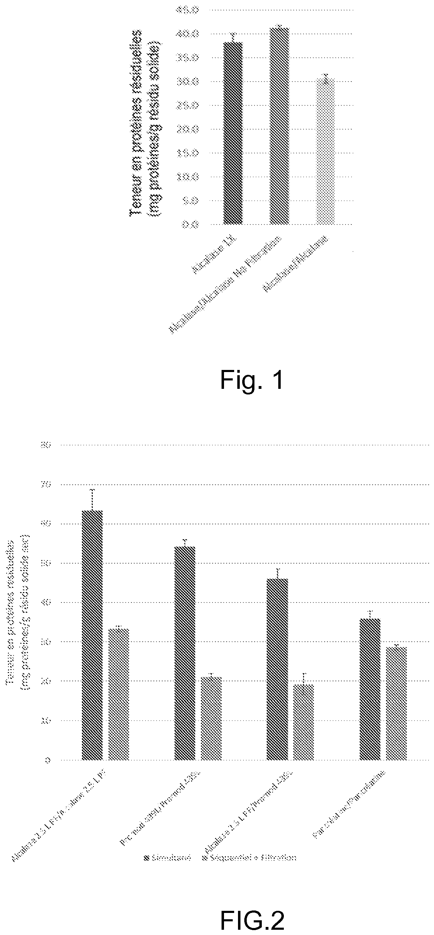

- **[****Fig 1****]** La figure 1 est un diagramme qui représente la teneur en protéines résiduelles de cuticules de *Tenebrio molitor* (en milligrammes de protéines par gramme de résidu solide) en fonction de trois procédés d'extraction différents mettant en oeuvre une hydrolyse enzymatique avec de l'Alcalase 2.5 L PF.
- **[****Fig 2****]** La figure 2 est un diagramme qui représente la teneur en protéines résiduelles de cuticules de *Tenebrio molitor* (en milligrammes de protéines par gramme de résidu solide sec) en fonction de deux procédés d'extraction différents (simultané ou séquentiel + filtration) mettant en oeuvre une hydrolyse enzymatique avec différentes endopeptidases à sérine.

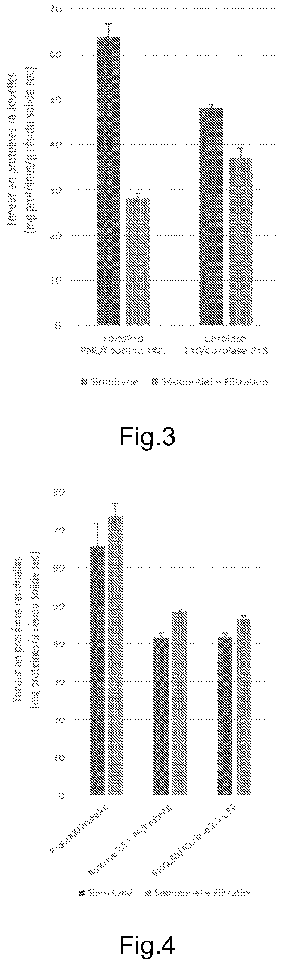

- **[****Fig 3****]** La figure 3 est un diagramme qui représente la teneur en protéines résiduelles de cuticules de *Tenebrio molitor* (en milligrammes de protéines par gramme de résidu solide sec) en fonction de deux procédés d'extraction différents (simultané ou séquentiel + filtration) mettant en oeuvre une hydrolyse enzymatique avec différentes métalloendopeptidases.
- **[****Fig 4****]** La figure 4 est un diagramme qui représente la teneur en protéines résiduelles de cuticules de *Tenebrio molitor* (en milligrammes de protéines par gramme de résidu solide sec) en fonction de deux procédés d'extraction différents (simultané ou séquentiel + filtration) mettant en oeuvre une hydrolyse enzymatique avec une protéase comprenant uniquement une exopeptidase (ProteAX) et/ou une protéase comprenant uniquement une endopeptidase à sérine (Alcalase 2.5 L PF).

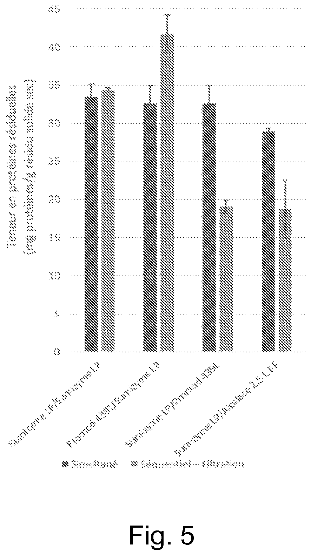

- **[****Fig 5****]** La figure 5 est un diagramme qui représente la teneur en protéines résiduelles de cuticules de *Tenebrio molitor* (en milligrammes de protéines par gramme de résidu solide sec) en fonction de deux procédés d'extraction différents (simultané ou séquentiel + filtration) mettant en oeuvre une hydrolyse enzymatique avec une protéase comprenant un mélange d'endopeptidases et d'une exopeptidase (Sumizyme LP) et/ou une protéase comprenant uniquement une endopeptidase à sérine (Promod 439L ou Alcalase 2.5 L PF).

### Exemple 1 : Obtention de cuticules broyées d'insectes

Des larves vivantes de *Tenebrio molitor,* ayant reçu un régime alimentaire à base de coproduits céréaliers (de type son de blé), sont convoyées en couche d'épaisseur comprise entre 2 et 10 cm, sur un tapis à bande perforée (1mm) jusqu'à une chambre de blanchiment. Les larves sont ainsi blanchies à l'eau à 92°C (buses d'aspersion) pendant 5 min. La température des larves en sortie de blanchiment est comprise entre 75°C et 92°C.

Une fois blanchies, les larves sont convoyées jusqu'à la trémie d'alimentation d'un séparateur à bande (séparateur à bande 601 de la société Baader), afin de séparer les cuticules de la partie molle des larves. Le diamètre des perforations du tambour est de 1,3 mm. La séparation s'effectue immédiatement après l'abattage de manière à ce que les larves n'aient pas le temps de refroidir à température ambiante. La partie molle des insectes est récupérée dans une cuve et les cuticules sont récupérées à l'aide d'un couteau racleur.

Les cuticules sont alors broyées en présence d'eau (dans un ratio massique des cuticules par rapport à l'eau de 1:6, par exemple 1200 mL d'eau pour 200 g de cuticules) à l'aide d'un Thermomix Vorwerk pendant 1 minute à la vitesse 10, ce qui conduit à des cuticules broyées ayant une taille d'environ 1 à 2 mm. Elles sont ensuite filtrées sur un Büchner ayant un filtre métallique avec une taille de maille de 500 µm, et lavées avec de l'eau (même volume d'eau que celui utilisé pour le broyage, soit 1200 mL d'eau dans cet exemple).

Enfin, les cuticules lavées sont pressées (Angelia 7500, Angel) afin d'éliminer l'excès d'eau pour obtenir une teneur en matière sèche de 52% ±5 en poids.

### Exemple 2 : Effet de la filtration entre deux extractions enzymatiques avec une protéase d'origine bactérienne - Alcalase 2.5 L PF

L'Alcalase 2.5 L PF est une endopeptidase à sérine issue de *Bacillus licheniformis* et commercialisée par Novozymes.

260 U d'Alcalase 2.5 L PF au total sont ajoutées à un mélange de 8,6 g de cuticules (poids humide) broyées obtenues selon le procédé décrit à l'Exemple 1, et de 86 g d'eau du robinet, selon trois procédés différents. Le pH du milieu réactionnel avant l'ajout de protéase dans ces trois procédés est de 7,3. Les trois procédés sont réalisés à une température de 55°C et les résultats sont indiqués à la Figure 1 :
- « Alcalase 1X » : les 260 U d'Alcalase sont ajoutées en une fois et la réaction d'hydrolyse est laissée pendant 5h.
- « Alcalase/Alcalase No filtration » : 130 U d'Alcalase sont ajoutées, la première hydrolyse est laissée pendant 2h, puis 130 U d'Alcalase sont à nouveau ajoutées et la seconde hydrolyse est laissée pendant 3h.
- « Alcalase/Alcalase » : 130 U d'Alcalase sont ajoutées, la première hydrolyse est laissée pendant 2h et le milieu réactionnel est alors filtré (filtre Büchner avec un maillage métallique 160 µm). Les cuticules récupérées sont ensuite mélangées avec 86 g d'eau du robinet et 130 U d'Alcalase sont à nouveau ajoutées, puis la seconde hydrolyse est laissée pendant 3h.

Après inactivation de l'enzyme pendant 15 min à 85°C, les milieux réactionnels sont filtrés (filtre Büchner avec un maillage métallique 160 µm).

La teneur en protéines résiduelles est déterminée par la méthode BCA (pour BiCinchoninic acid Assay), en utilisant 2 mg de résidu solide sec. Le milieu réactionnel obtenu à l'issue de la méthode BCA est dilué avec de l'eau par un facteur de dilution de 4 avant la mesure au spectrophotomètre.

Ainsi, pour une même quantité totale de protéase ajoutée, la déprotéinisation est légèrement moins efficace lorsque deux hydrolyse sont réalisées successivement et sans filtration en comparaison avec une seule hydrolyse (respectivement 41 mg et 38 mg de protéines résiduelles par gramme de résidu solide).

En revanche, lorsque deux hydrolyse sont réalisées successivement et avec filtration entre les deux hydrolyses, le taux de déprotéinisation est significativement augmenté en comparaison à l'hydrolyse réalisée en une seule fois (respectivement 31 mg et 38 mg de protéines résiduelles par gramme de résidu solide).

Par conséquent, une étape de filtration entre deux hydrolyses successives a pour effet d'augmenter le taux de déprotéinisation des cuticules d'insectes, et donc la pureté de la chitine résultante.

### Exemple 3 : Procédé selon l'invention et procédé comparatif - endopeptidase à sérine

Les endopeptidases à sérine mises en oeuvre dans cet Exemple 3 sont la subtilisine et la pancréatine. La subtilisine provient de deux fournisseurs différents : Alcalase 2.5 L PF (Novozymes) et Promod 439L (Biocatalysts), et est issue de *Bacillus licheniformis.* La pancréatine provient de Sigma-Aldrich et est issue de Sus *scrofa domesticus.*

Deux procédés différents ont été utilisés : simultané (procédé comparatif) et séquentiel + filtration (procédé selon l'invention), dont les résultats sont présentés en Figure 2.

### • Procédé simultané

L'ajout de protéase se fait sur un mélange de 2 g de cuticules (poids sec) broyées, obtenues selon le procédé décrit à l'Exemple 1, avec 39,8 g d'eau du robinet à un pH de 7,3.

Le procédé simultané a été réalisé en ajoutant 40 U au total de protéase(s) par gramme de cuticules (poids sec) au mélange de cuticules et d'eau, pour un temps de réaction de 4h et une température dépendant de la protéase mise en oeuvre.

Alcalase 2.5 L PF / Alcalase 2.5 L PF : une seule étape d'hydrolyse avec 40 U d'Alcalase 2.5 L PF par gramme de cuticules (poids sec) et à une température de 55°C.

Promod 439L / Promod 439L : une seule étape d'hydrolyse avec 40 U de Promod 439L par gramme de cuticules (poids sec) et à une température de 57°C.

Alcalase 2.5 L PF / Promod 439L : une seule étape d'hydrolyse avec 20 U d'Alcalase 2.5 L PF et avec 20 U de Promod 439L par gramme de cuticules (poids sec), et à une température de 56°C.

Pancréatine/Pancréatine : une seule étape d'hydrolyse avec 40 U de Pancréatine par gramme de cuticules (poids sec) et à une température de 45°C.

### • Procédé séquentiel + filtration

L'ajout de protéase se fait sur un mélange de 2 g de cuticules (poids sec) broyées et lavées, obtenues selon le procédé décrit à l'Exemple 1, avec 39,8 g d'eau du robinet à un pH de 7,3.

Le procédé séquentiel + filtration a également été réalisé en ajoutant 40 U au total de protéases par gramme de cuticules (poids sec) au mélange de cuticules et d'eau, pour un temps de réaction total de 4h et une température dépendant de la protéase mise en oeuvre. Toutefois, dans ce procédé, une première hydrolyse est réalisée avec 20 U de protéase par gramme de cuticules (poids sec) pendant 2h, puis le milieu d'hydrolyse est filtré (maillage 160 µm) ; les cuticules hydrolysées récupérées sont alors à nouveau mélangées avec 39,8 g d'eau du robinet à un pH de 7,3 et une seconde hydrolyse est à nouveau effectuée avec 20 U de protéase par gramme de cuticules (poids sec) pendant 2h.

Alcalase 2.5 L PF / Alcalase 2.5 L PF : première hydrolyse avec 20 U d'Alcalase 2.5 L PF par gramme de cuticules (poids sec) à une température de 55°C, puis filtration du milieu d'hydrolyse, et seconde hydrolyse avec 20 U d'Alcalase 2.5 L PF par gramme de cuticules (poids sec) à une température de 55°C.

Promod 439L / Promod 439L : première hydrolyse avec 20 U de Promod 439L par gramme de cuticules (poids sec) à une température de 57°C, puis filtration du milieu d'hydrolyse, et seconde hydrolyse avec 20 U de Promod 439L par gramme de cuticules (poids sec) à une température de 57°C.

Alcalase 2.5 L PF / Promod 439L : première hydrolyse avec 20 U d'Alcalase 2.5 L PF par gramme de cuticules (poids sec) à une température de 55°C, puis filtration du milieu d'hydrolyse, et seconde hydrolyse avec 20 U de Promod 439L par gramme de cuticules (poids sec) à une température de 57°C.

Pancréatine/Pancréatine : première hydrolyse avec 20 U de Pancréatine par gramme de cuticules (poids sec) à une température de 45°C, puis filtration du milieu d'hydrolyse, et seconde hydrolyse avec 20 U de Pancréatine par gramme de cuticules (poids sec) à une température de 45°C.

Dans les deux procédés (simultané ou séquentiel + filtration), la protéase est ensuite inactivée pendant 15 min à 85°C, puis le milieu réactionnel est filtré (maillage 160 µm).

La teneur en protéines résiduelles est déterminée par la méthode BCA (pour BiCinchoninic acid Assay), en utilisant 2 mg de résidu solide sec. Le milieu réactionnel obtenu à l'issue de la méthode BCA est dilué avec de l'eau par un facteur de dilution de 4 avant la mesure au spectrophotomètre.

La Figure 2 montre que, lorsque deux hydrolyses successives sont réalisées avec la protéase et avec filtration entre les deux hydrolyses (séquentiel + filtration), le taux de déprotéinisation est significativement augmenté en comparaison à l'hydrolyse réalisée en une seule fois (simultané), et ce pour les trois protéases testées (voir Alcalase 2.5 L PF / Alcalase 2.5 L PF, Promod 439L / Promod 439L et Pancréatine / Pancréatine), ou pour une alternance de protéases (voir Alcalase 2.5 L PF / Promod 439L).

Par conséquent, une étape de filtration entre deux hydrolyses avec une endopeptidase à sérine a pour effet d'augmenter significativement le taux de déprotéinisation des cuticules d'insectes, et donc la pureté de la chitine résultante.

### Exemple 4 : Procédé selon l'invention et procédé comparatif-métalloendopeptidase

Les métalloendopeptidases mises en oeuvre dans cet Exemple 4 sont la bacillolysine et la thermolysine. La bacillolysine est FoodPro PNL qui provient de Dupont Danisco et est issue de *Bacillus amyloliquefaciens.* La thermolysine est Corolase 2TS qui provient de AB Enzymes et est issue de *Bacillus stearothermophilus.*

Les procédés simultané et séquentiel + filtration de l'Exemple 3 ont été reproduits avec ces métalloendopeptidases et à une température de 50°C pour la FoodPro PNL et 60°C pour Corolase 2TS. Les résultats sont présentés en Figure 3.

La Figure 3 montre que, lorsque deux hydrolyses successives sont réalisées avec FoodPro PNL ou Corolase 2TS et avec filtration entre les deux hydrolyses (séquentiel + filtration), le taux de déprotéinisation est significativement augmenté en comparaison à l'hydrolyse réalisée en une seule fois (simultané).

Par conséquent, une étape de filtration entre deux hydrolyses avec une métalloendopeptidase a pour effet d'augmenter significativement le taux de déprotéinisation des cuticules d'insectes, et donc la pureté de la chitine résultante.

### Exemple 5 : Influence de la nature de la protéase : nécessité de la présence d'une endopeptidase lors de chaque étape d'hydrolyse

Afin d'étudier l'influence de la nature endo- ou exopeptidase sur le taux de déprotéinisation, une protéase comprenant principalement de l'aminopeptidase (exopeptidase) a été mise en oeuvre (ProteAX issue *d'Aspergillus oryzae* et vendu par Amano Enzyme ; numéro IUB déclaré par le fournisseur : 3.4.11.1), ainsi que de la subtilisine (Alcalase 2.5 L PF).

Les procédés simultané et séquentiel + filtration de l'Exemple 3 ont été reproduits, les résultats étant présentés en Figure 4 :
- ProteAX / ProteAX : la température est de 50°C.
- Alcalase 2.5 L PF / ProteAX : pour le procédé simultané, la température est de 52,5°C ; pour le procédé séquentiel, Alcalase 2.5 L PF est ajoutée en premier à une température de 55°C, et ProteAX est ajoutée en deuxième à une température de 50°C.
- ProteAX / Alcalase 2.5 L PF : pour le procédé simultané, la température est de 52,5°C ; pour le procédé séquentiel, ProteAX est ajoutée en premier à une température de 50°C, et Alcalase 2.5 L PF est ajoutée en deuxième à une température de 55°C.

La Figure 4 montre que, lorsque deux hydrolyses successives sont réalisées avec ProteAX et avec filtration entre les deux hydrolyses, le taux de déprotéinisation est significativement diminué en comparaison à l'hydrolyse réalisée en une seule fois. En outre, le fait que deux hydrolyses successives soit réalisées avec ProteAX avant ou après Alcalase 2.5 L PF ne permet pas non plus d'augmenter le taux de déprotéinisation par rapport à une hydrolyse réalisée en une fois avec les deux protéases (ajout simultané) : l'utilisation de ProteAX dans un procédé séquentiel + filtration diminue donc significativement le taux de déprotéinisation.

Par conséquent, dans le procédé selon l'invention, la présence d'une endopeptidase est nécessaire lors de chaque étape d'hydrolyse afin d'augmenter le taux de déprotéinisation.

### Exemple 6 : Influence de la nature de la protéase lors de la seconde hydrolyse enzymatique : nécessité d'une protéase ne comprenant pas une exopeptidase lors de la seconde étape d'hydrolyse

Afin d'étudier l'influence de l'utilisation d'une protéase comprenant à la fois des endopeptidases et une exopeptidase sur le taux de déprotéinisation, une protéase comprenant un mélange d'oryzine, d'aspergillopepsine I (endopeptidases) et de leucyl aminopeptidase (exopeptidase) a été mise en oeuvre (Sumizyme LP issue *d'Aspergillus oryzae* et vendu par Shin Nihon Chemical), optionnellement en alternance avec de la subtilisine (Alcalase 2.5 L PF et Promod 439L).

Les procédés simultané et séquentiel + filtration de l'Exemple 3 ont été reproduits, les résultats étant présentés en Figure 5 :
- Sumizyme LP / Sumizyme LP : la température est de 45°C.
- Promod 439L / Sumizyme LP : pour le procédé simultané, la température est de 50°C ; pour le procédé séquentiel, Promod 439L est ajoutée en premier à une température de 57°C, et Sumizyme LP est ajoutée en deuxième à une température de 45°C.
- Sumizyme LP / Promod 439L : pour le procédé simultané, la température est de 50°C ; pour le procédé séquentiel, Sumizyme LP est ajoutée en premier à une température de 45°C, et Promod 439L est ajoutée en deuxième à une température de 57°C.
- Sumizyme LP / Alcalase 2.5 L PF : pour le procédé simultané, la température est de 50°C ; pour le procédé séquentiel, Sumizyme LP est ajoutée en premier à une température de 45°C, et Alcalase 2.5 L PF est ajoutée en deuxième à une température de 55°C.

Lorsque deux hydrolyses successives sont réalisées avec Sumizyme LP et avec filtration entre les deux hydrolyses, le taux de déprotéinisation est similaire à l'hydrolyse réalisée en une seule fois. De plus, une seconde hydrolyse avec Sumizyme LP après une première hydrolyse avec Promod 439L suivie d'une filtration diminue significativement le taux de déprotéinisation par rapport à une hydrolyse réalisée en une seule fois.

En revanche, si la première hydrolyse est réalisée avec Sumizyme LP et la seconde avec Promod 439L dans le procédé séquentiel + filtration (Sumizyme LP / Promod 439L), alors le taux de déprotéinisation est significativement augmenté en comparaison avec l'hydrolyse réalisée en une seule fois. De même lorsque Promod 439L est remplacé par Alcalase 2.5 L PF (Sumizyme LP / Alcalase 2.5 L PF).

Par conséquent, lorsqu'une filtration est effectuée entre deux hydrolyses enzymatiques, il n'est pas nécessaire que la protéase mise en oeuvre lors de la première hydrolyse ne comprenne pas d'exopeptidase. En revanche, cette condition est impérative pour la protéase mise en oeuvre lors de la seconde hydrolyse, et permet d'augmenter significativement le taux de déprotéinisation.

## Revendications

1. Procédé d'obtention de chitine à partir de cuticules d'insectes comprenant :
- une première hydrolyse enzymatique des cuticules d'insectes mettant en oeuvre au moins une endopeptidase,
- la séparation des cuticules hydrolysées résultant de la première hydrolyse enzymatique, du milieu d'hydrolyse,
- une seconde hydrolyse enzymatique des cuticules hydrolysées mettant en oeuvre au moins une endopeptidase, à l'exclusion d'exopeptidase.

2. Procédé selon la revendication 1, dans lequel la au moins une endopeptidase mise en oeuvre lors de la seconde hydrolyse enzymatique est une endopeptidase ou un mélange d'endopeptidases choisie(s) parmi les endopeptidases à sérine, les endopeptidases à cystéine, les protéases aspartiques, les métalloendopeptidases et les endopeptidases à thréonine.

3. Procédé selon la revendication 1 ou 2, dans lequel la au moins une endopeptidase mise en oeuvre lors de la seconde hydrolyse enzymatique est une endopeptidase à sérine ou un mélange d'endopeptidases à sérine, et/ou une métalloendoprotéase ou un mélange de métalloendoprotéases, chaque endopeptidase à sérine étant choisie parmi le groupe constitué par la subtilisine et la trypsine, chaque métalloendoprotéase étant choisie parmi le groupe constitué par la bacillolysine et la thermolysine.

4. Procédé selon la revendication 1 ou 2, dans lequel la au moins une endopeptidase mise en oeuvre lors de la seconde hydrolyse enzymatique est d'origine bactérienne.

5. Procédé selon l'une quelconque des revendications 1 à 4, comprenant en outre une étape de broyage des cuticules d'insectes préalable à la première hydrolyse enzymatique.

6. Procédé selon l'une quelconque des revendications 1 à 5, dans lequel les cuticules d'insectes sont obtenues par une étape de séparation des cuticules de la partie molle des insectes.

7. Procédé selon l'une quelconque des revendications 1 à 6, dans lequel la durée totale des étapes d'hydrolyse est inférieure à 6 h.

8. Procédé selon l'une quelconque des revendications 1 à 7, dans lequel la quantité totale desdites au moins une endopeptidase mises en oeuvre est comprise entre 10 U et 500 U par gramme de cuticules d'insectes, en poids sec, et dans lequel « U », aussi appelé unité enzymatique, représente la quantité d'enzyme nécessaire pour libérer une micromole de tyrosine (provenant de la caséine) en une minute, à 37°C et à pH 7,5.

9. Procédé selon l'une quelconque des revendications 1 à 8, comprenant en outre, à l'issue de la seconde hydrolyse, une étape de traitement chimique.

10. Procédé d'obtention de chitosan comportant un procédé d'obtention de chitine selon l'une quelconque des revendications 1 à 9, et comprenant en outre une étape de désacétylation de la chitine.

## Patentansprüche

1. Verfahren zum Gewinnen von Chitin aus der Nagelhaut von Insekten, umfassend:
- eine erste enzymatische Hydrolyse der Insektennagelhaut unter Verwendung von mindestens einer Endopeptidase,
- Abtrennen der aus der ersten enzymatischen Hydrolyse resultierenden hydrolysierten Nagelhaut vom Hydrolysemedium,
- eine zweite enzymatische Hydrolyse der hydrolysierten Nagelhaut unter Verwendung von mindestens einer Endopeptidase, ausgenommen Exopeptidase.

2. Verfahren nach Anspruch 1, wobei die mindestens eine Endopeptidase, die während der zweiten enzymatischen Hydrolyse verwendet wird, eine Endopeptidase oder eine Mischung von Endopeptidasen ist, die aus Serin-Endopeptidasen, Cystein-Endopeptidasen, Asparagin-Proteasen, Metalloendopeptidasen und Threonin-Endopeptidasen ausgewählt ist.

3. Verfahren nach Anspruch 1 oder 2, wobei die mindestens eine Endopeptidase, die während der zweiten enzymatischen Hydrolyse verwendet wird, eine Serin-Endopeptidase oder eine Mischung aus Serin-Endopeptidasen und/oder eine Metalloendoprotease oder eine Mischung aus Metalloendoproteasen ist, wobei jede Serin-Endopeptidase aus der Gruppe bestehend aus Subtilisin und Trypsin ausgewählt ist, wobei jede Metalloendoprotease aus der Gruppe bestehend aus Bacillolysin und Thermolysin ausgewählt ist.

4. Verfahren nach Anspruch 1 oder 2, wobei die mindestens eine Endopeptidase, die während der zweiten enzymatischen Hydrolyse verwendet wird, bakteriellen Ursprungs ist.

5. Verfahren nach einem der Ansprüche 1 bis 4, das ferner einen Schritt des Zerkleinerns der Insektennagelhaut vor der ersten enzymatischen Hydrolyse umfasst.

6. Verfahren nach einem der Ansprüche 1 bis 5, wobei die Insektennagelhaut durch einen Schritt des Trennens der Nagelhaut vom Weichgewebe der Insekten erhalten wird.

7. Verfahren nach einem der Ansprüche 1 bis 6, wobei die Gesamtzeit der Hydrolyseschritte weniger als 6 Stunden beträgt.

8. Verfahren nach einem der Ansprüche 1 bis 7, wobei die Gesamtmenge der mindestens einen verwendeten Endopeptidase zwischen 10 U und 500 U pro Gramm Insektennagelhaut, bezogen auf das Trockengewicht, beträgt, und wobei "U", das ebenfalls als Enzymeinheit bezeichnet wird, für die Menge an Enzym steht, die benötigt wird, um ein Mikromol Tyrosin (aus Kasein) innerhalb einer Minute bei 37 °C und einem PH-Wert von 7,5 freizusetzen.

9. Verfahren nach einem der Ansprüche 1 bis 8, das ferner einen Schritt des chemischen Behandelns nach der zweiten Hydrolyse umfasst.

10. Verfahren zum Gewinnen von Chitosan, das ein Verfahren zum Gewinnen von Chitin nach einem der Ansprüche 1 bis 9 enthält und ferner einen Schritt des Deacetylierens des Chitins umfasst.

## Claims

1. Method for obtaining chitin from insect cuticles comprising:
- performing a first enzymatic hydrolysis of insect cuticles using at least one endopeptidase,
- separating the hydrolyzed cuticles resulting from the first enzymatic hydrolysis, from the hydrolysis medium,
- performing a second enzymatic hydrolysis of the hydrolyzed cuticles using at least one endopeptidase, excluding exopeptidase.

2. Method according to claim 1, wherein the at least one endopeptidase used in the second enzymatic hydrolysis is an endopeptidase or a mixture of endopeptidases selected from serine endopeptidases, cysteine endopeptidases, aspartic proteases, metalloendopeptidases and threonine endopeptidases.

3. Method according to claim 1 or 2, wherein the at least one endopeptidase used in the second enzymatic hydrolysis is a serine endopeptidase or a mixture of serine endopeptidases, and/or a metalloendoprotease or a mixture of metalloendoproteases, each serine endopeptidase being selected from the group constituted by subtilisin and trypsin, each metalloendoprotease being selected from the group constituted by bacillolysin and thermolysin.

4. Method according to claim 1 or 2, wherein the at least one endopeptidase used in the second enzymatic hydrolysis is of bacterial origin.

5. Method according to any one of claims 1 to 4, further comprising a step of grinding the insect cuticles prior to the first enzymatic hydrolysis.

6. Method according to any one of claims 1 to 5, wherein the insect cuticles are obtained by a step of separating the cuticles from the soft part of the insects.

7. Method according to any one of claims 1 to 6, wherein the total time of the hydrolysis steps is less than 6 h.

8. Method according to any one of claims 1 to 7, wherein the total amount of said at least one endopeptidase used is between 10 U and 500 U per gram of insect cuticle, on a dry weight basis, and wherein "U", also referred to as an enzyme unit, represents the amount of enzyme required to release one micromole of tyrosine (from casein) in one minute at 37°C and pH 7.5.

9. Method according to any one of claims 1 to 8, further comprising, after the second hydrolysis, a chemical treatment step.

10. Method of obtaining chitosan comprising a method of obtaining chitin according to any one of claims 1 to 9, and further comprising a step of deacetylating the chitin.
